(19) Europäisches Patentamt
European Patent Office
Office européen des brevets

(11) Veröffentlichungsnummer: **0 033 095**
**B1**

(12) # EUROPÄISCHE PATENTSCHRIFT

(45) Veröffentlichungstag der Patentschrift: **19.10.83**

(51) Int. Cl.³: **C 07 D 257/06,** A 61 K 31/41

(21) Anmeldenummer: **81100260.9**

(22) Anmeldetag: **15.01.81**

(54) **5-Amino-tetrazol-Derivate von Retinsäuren, ihre Herstellung und diese enthaltende pharmazeutische Zubereitungen.**

(30) Priorität: **25.01.80 DE 3002545**

(43) Veröffentlichungstag der Anmeldung: **05.08.81 Patentblatt 81/31**

(45) Bekanntmachung des Hinweises auf die Patenterteilung: **19.10.83 Patentblatt 83/42**

(84) Benannte Vertragsstaaten: **AT BE CH DE FR GB IT LI LU NL SE**

(56) Entgegenhaltungen:
**EP-A-0 009 777**
**DE-A-2 102 586**
**DE-A-2 300 107**
**US-A-4 108 880**

(73) Patentinhaber: **BASF Aktiengesellschaft, Carl-Bosch-Strasse 38, D-6700 Ludwigshafen (DE)**

(72) Erfinder: **Frickel, Fritz-Frieder, Dr., Silvanerweg 7, D-6705 Deidesheim (DE)**
Erfinder: **Nuerrenbach, Axel, Dr., Koenigsberger Strasse 7, D-6718 Gruenstadt (DE)**

**EP 0 033 095 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# 5-Amino-tetrazol-Derivate von Retinsäuren, ihre Herstellung und diese enthaltende pharmazeutische Zubereitungen

Die Erfindung betrifft 5-Amino-tetrazol-Derivate von Retinsäuren und ihre physiologisch verträglichen Salze, Verfahren zu ihrer Herstellung und diese enthaltende pharmazeutische Zubereitungen, die insbesondere bei der Behandlung von Neoplasien, Akne, Dermatosen, Psoriasis, dermatologischen Affektionen und bei rheumatischen Erkrankungen verwendet werden können.

Es ist beispielsweise aus der DE-OS 2 102 586 bekannt, daß am Amidstickstoff gegebenenfalls substituierte Amide der all-E-Retinsäure pharmakologische Wirkungen bei der topischen und systemischen Therapie von Neoplasien, Akne, Psoriasis und anderen dermatologischen Affektionen aufweisen. Weiterhin gehen beispielsweise aus der DE-OS 2 300 107 all-E-Vitamin-A-säureamide mit höheren Alkylresten, mit cyclischen Amidresten und insbesondere substituierten Anilidresten für etwa den gleichen Verwendungszweck hervor.

In der US-PS 4 108 880 wird beschrieben, daß bestimmte Ester und substituierte Amide der all-E-Retinsäure wegen ihrer UV-Licht absorbierenden Eigenschaften als Sonnenschutzmittel verwendet werden können.

Dem Fachmann ist bekannt, daß die obengenannten Retinsäureamide in ihrer Wirkung nicht immer befriedigen. Nachteilig ist vor allen Dingen ihre zu geringe Wirkungsstärke, die diese Verbindungen als Mittel in der topischen und systemischen Therapie von Neoplasien, Akne, Psoriasis und anderen dermatologischen Affektionen wenig geeignet erscheinen lassen. Es überrascht somit nicht, daß keine der in der DE-OS 2 102 586 oder DE-OS 2 300 107 beschriebenen Verbindungen tatsächlich als Arzneimittel verwendet wird. Dagegen hat die Vitamin-A-säure bekanntlich zu einem Handelspräparat, z. B. für die Bekämpfung von Akne, geführt, wobei die weiter unten aufgeführten Nachteile in Kauf genommen werden müssen.

Der vorliegenden Erfindung liegt daher die Aufgabe zugrunde, wirksamere Retinoide zur Verfügung zu stellen.

Es wurde gefunden, daß Verbindungen der Formel (I),

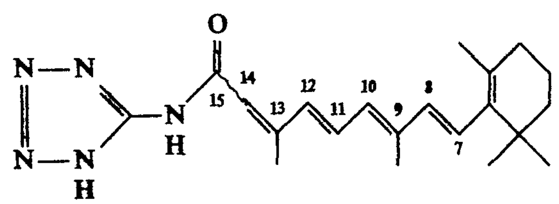

(I)

in der die geschweifte Bindung zwischen den Kohlenstoffatomen 14 und 15 eine all-E- oder 13-Z-Verknüpfung bedeutet, und ihre physiologisch verträglichen Salze wertvolle pharmakologische Eigenschaften aufweisen.

Dementsprechend sind als erfindungsgemäße Verbindungen das all-E-Retinsäure-N-(tetrazol-5-yl)-amid und das 13-Z-Retinsäure-N-(tetrazol-5-yl)-amid zu nennen.

Diese können zur topischen und systemischen Therapie von benignen und malignen Neoplasien, von praemalignen Läsionen, sowie ferner zur systemischen und topischen Prophylaxe der genannten Affektionen verwendet werden. Sie sind darüber hinaus für die topische und systemische Therapie von Akne, Psoriasis und anderer mit einer verstärkten oder pathologisch veränderten Verhornung einhergehenden Dermatosen, wie auch von entzündlichen und allergischen dermatologischen Affektionen oder von Erkrankungen, wie sie beispielsweise bei G. Plewig und A. M. Kligman in »Acne-Morphogenesis and Treatment«, Springer-Verlag, Heidelberg 1975, beschrieben werden, geeignet. Weiterhin können die erfindungsgemäßen Retinoide gegebenenfalls auch bei der Bekämpfung von Schleimhauterkrankungen mit entzündlichen oder degenerativen, metaplastischen Veränderungen oder von rheumatischen Erkrankungen in Betracht kommen.

In einer kürzlich erfolgten epidemiologischen Studie (J. Natl. Cancer Inst. 62, 1435—1438 [1979]) wird beschrieben, daß Vitamin-A das Risiko der Entstehung von Lungenkrebs mindert. Für Retinoide, den chemischen Analogen zu Vitamin A, wurde schon vor einigen Jahren eine chemopreventive Wirkung bei der Carcinogenese im Tierversuch belegt und beispielsweise von M. B. Sporn, »Chemoprevention of Cancer with Retinoids« in Federation Proceedings, Vol. 38, 2528—2534 (1979) zusammenfassend dargestellt. In dieser Publikation wird eindrucksvoll dargelegt, daß eine Tumorprophylaxe mit Retinoiden wirksamere und weniger toxische Verbindungen erfordert. All-trans-Retinsäure, als eine der bisherigen aktivsten Verbindung unter den Retinoiden zur Inhibierung praekanzeroser Prozesse, scheidet wegen ihrer nicht-tolerierbaren toxischen Nebenwirkungen für die praktische Anwendung aus. Über die toxischen Effekte der Retinsäure berichtet beispielsweise S. D. Harrison in Nature, Band 269, 1977, auf den Seiten 511—512, daß bereits geringfügig erhöhte Dosen zu schweren Schädigungen des Organismus führen, wie sie sich uns beispielsweise im Krankheitsbild der Hypervitaminose-A darstellen.

Durch die erfindungsgemäßen Verbindungen der Formel (I) können die obengenannten Nachteile

vermieden werden, da diese bei vergleichbar niederer zellularer Toxizität und einer hohen pharmakologischen Aktivität einen besonders günstigen therapeutischen Index aufweisen.

Die praktische Nützlichkeit von Retinoiden bei der pharmakologischen Anwendung hängt jedoch keineswegs nur von der pharmakologischen Aktivität und der sie begleitenden Toxizität ab. Entscheidend ist in hohem Maße das pharmakokinetische Verhalten der einzelnen Wirkstoffe. Beispielsweise ist es für die Anwendung eines Retinoids nachteilig, wenn es im Verlaufe der Behandlung durch den Stoffwechsel in einzelnen Organen in so hohen Konzentrationen gespeichert wird, daß toxische Nebenwirkungen auftreten. Andererseits kann die Konzentration eines Retinoids oder seines aktiven Metaboliten in bestimmten von Praekanzerosen oder Karzinomen befallenen Organen so gering sein, daß die erwünschte Heilwirkung dort nicht erreicht werden kann. Aus diesem Grund scheidet beispielsweise Retinol oder Retinylacetat für die Therapie aus, da in beiden Fällen eine unerträglich hohe Retinoidkonzentration in der Leber zu schweren irreversiblen Organschädigungen führt.

Derartige Nachteile können durch die erfindungsgemäßen Verbindungen vermieden werden, da sie bei mehrmaliger Applikation zu einer erwünschten, organspezifischen Erhöhung der Retinoidkonzentration führen, ohne daß dies mit einer merklichen Steigerung der Retinoidkonzentration in der Leber einhergeht.

Es wird darauf hingewiesen, daß die durch die Formel (I) dargestellten Verbindungen ein tautomeres Wasserstoffatom aufweisen, und daß die Verbindungen somit in der 1H-Tetrazol-5-yl-Form (Formel I) und/oder der 2H-Tetrazol-5-yl-Form (Formel Ia) vorliegen können.

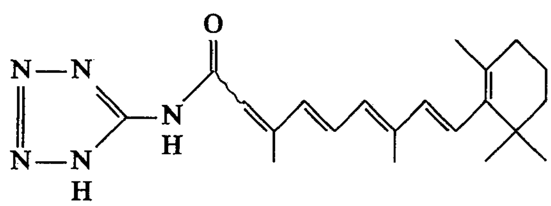

(I)

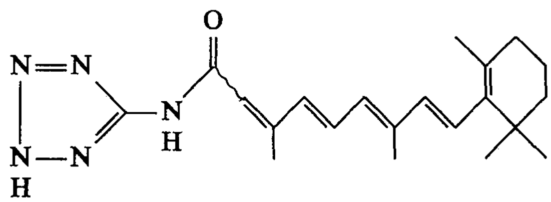

(Ia)

Die Erfindung umfaßt beide Formen; aus Gründen der Einfachheit wird die Formel (I) gewählt.

Die erfindungsgemäßen Verbindungen der Formel (I) werden hergestellt, indem man ein reaktionsfähiges Retinsäurederivat der Formel (II)

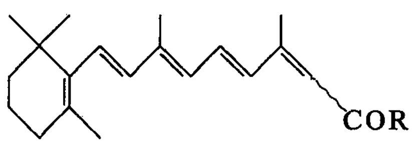

(II)

in der die geschweifte Bindung zwischen den Kohlenstoffatomen 14 und 15 eine all-E- oder 13-Z-Verknüpfung bedeutet und R für eine geeignete Abgangsgruppe steht, zweckmäßigerweise in einem Lösungsmittel und ggf. in Gegenwart eines säurebindenden Mittels in an sich bekannter Weise mit 5-Aminotetrazol umsetzt und die erhaltene Verbindung ggf. mit einer basischen Substanz in ein physiologisch verträgliches Salz überführt.

Die Abgangsgruppe R stellt bevorzugt ein Halogenatom, insbesondere Chlor oder Brom, vorzugsweise Chlor, oder den N-Oxy-succinimid-Rest dar.

Die Umsetzungen werden bei Temperaturen von −40° C bis 50° C durchgeführt. Die Umsetzungen können unter Atmosphärendruck oder in einem geschlossenen Gefäß unter erhöhtem Druck durchgeführt werden.

Zweckmäßigerweise werden die Umsetzungen in Gegenwart eines inerten Verdünnungs- oder Lösungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol oder eines Alkylbenzols, wie Toluol oder Xylol, oder eines gesättigten aliphatischen Kohlenwasserstoffs, wie Hexan, Heptan oder Isooctan, eines niederen aliphatischen Ketons, wie Aceton, Methylethylketon oder Methylisobutylketon, eines Dialkylformamids, wie Dimethyl- oder Diethylformamid, oder in Mischungen der genannten Lösungsmittel durchgeführt.

Bevorzugte Lösungsmittel bei der Umsetzung eines Retinsäure-chlorids gemäß Formel II mit

5-Amino-tetrazol sind Ether, insbesondere Diethylether und Tetrahydrofuran, sowie Dialkylformamide, insbesondere Dimethylformamid, wobei die Umsetzung bevorzugt bei Temperaturen von −20° C bis 30° C durchgeführt wird.

Bevorzugt werden die Umsetzungen bei Verwendung von Retinsäurehalogeniden in Gegenwart einer Base als säurebindendem Mittel vorgenommen. Geeignete Basen sind Alkalimetallcarbonate, -hydrogencarbonate, insbesondere des Natriums und Kaliums, organische tertiäre Basen, wie Pyridin oder niedere Trialkylamine, wie Trimethyl- oder Triethylamin. Dabei wird die verwendete Base im Verhältnis zum eingesetzten Retinsäurehalogenid in stöchiometrischer Menge oder in geringem Überschuß verwendet.

Weiterhin werden die erfindungsgemäßen Verbindungen der Formel (I) hergestellt, indem man all-E- oder 13-Z-Retinsäure in Gegenwart eines die Carboxylgruppe aktivierenden, wasserspaltenden Mittels in einem Lösungsmittel mit 5-Aminotetrazol kondensiert und ggf. die erhaltene Verbindung mit einer Base in ein physiologisch verträgliches Salz überführt.

Als wasserabspaltende aktivierende Reagentien können die üblicherweise bei der Peptidsynthese verwendeten Reagentien verwendet werden, wie sie beispielsweise von Schröder und Lübke in »The Peptides«, Band I, Academic Press, N. Y., 1965, Seiten 77 bis 128, beschrieben werden. Das allgemeine Prinzip der Synthese besteht in der Aktivierung der Carboxylgruppe, beispielsweise durch Behandlung mit einem Carbodiimid, wie N,N'-Dicyclohexylcarbodiimid, oder durch intermediäre Bildung des Säureazids, eines gemischten Anhydrids (beispielsweise mit Kohlensäuremonoestern), eines aktivierten Esters (beispielsweise des p-Nitrophenylesters) oder eines heterocyclischen Amids (beispielsweise eines Imidazolids) der entsprechenden Retinsäure.

Die Behandlung einer an der Carboxylgruppe aktivierten Verbindung mit 5-Aminotetrazol führt zu den erfindungsgemäßen Verbindungen. Die Aktivierungs- und Verknüpfungsreaktionen werden in inerten Lösungsmitteln durchgeführt, vorzugsweise in N,N-Dimethylformamid, Tetrahydrofuran, Dioxan, Methylenchlorid, Nitromethan, Acetonitril, Dimethylsulfoxid, N,N-Dimethylacetamid und Hexamethylenphosphorsäuretriamid.

Die Umsetzungen erfolgen bei Temperaturen von 20 bis 100° C für beide Stufen, d. h. bei der Reaktion der Säure mit dem Kupplungsmittel und bei der Reaktion des aktivierten Zwischenprodukts mit 5-Aminotetrazol. Die Kondensationsreaktion kann entweder stufenweise durchgeführt werden, indem man das aktivierte Zwischenprodukt vor der Zugabe des 5-Aminotetrazols isoliert oder indem man zweckmäßigerweise die Reaktionspartner nacheinander ohne Isolierung von Zwischenstufen zur Reaktion bringt.

Bei einer bevorzugten Verknüpfungsmethode verwendet man das N,N-Carbonyldiimidazol.

Diese bevorzugte Reaktion wird durchgeführt, indem man in einem der o. g. inerten Lösungsmittel mit dem 5-Aminotetrazol umsetzt, unabhängig davon, ob in zweistufiger oder einstufiger Verfahrensweise gearbeitet wird. Das bevorzugte Lösungsmittel ist N,N-Dimethylformamid, die bevorzugte Reaktionstemperatur liegt bei beiden Stufen bei Temperaturen von 20 bis 60° C.

Das Zwischenprodukt all-E-Retinsäureimidazolid wird beispielsweise von Staab und Bräunling in Liebigs Annalen der Chemie, Band 654, Seite 129 (1962), beschrieben.

Es sei erwähnt, daß als Ausgangsverbindungen der Formel (II) gegebenenfalls auch Mischungen der beiden all-E- und 13-Z-Isomeren in Betracht kommen, da bei der technischen Herstellung dieser Verbindungen Gemische unter Umständen entstehen können. Die in solchen Fällen resultierende Mischung der erfindungsgemäßen Verbindungen der Formel (I) können durch die HPLC-Analyse oder durch ein $^{13}C$-NMR-Spektrum quantitativ bestimmt und gegebenenfalls durch fraktionierende Kristallisation oder Chromatographie mit beispielsweise Kieselgelsäulen oder durch präparative HPL-Chromatographie isomerenrein isoliert werden.

Die erfindungsgemäßen Verbindungen weisen ein acides Wasserstoffatom auf und können daher mit Basen in an sich üblicher Weise in ein physiologisch verträgliches, gut wasserlösliches Salz überführt werden. Geeignete Salze sind beispielsweise Ammonium-, Alkalimetall-, insbesondere des Natriums, Kaliums und Lithiums, Erdalkalimetallsalze, insbesondere des Calciums oder Magnesiums, sowie Salze mit geeigneten organischen Basen, wie mit niederen Alkylaminen, z. B. Methylamin oder Ethylamin, mit substituierten niederen Alkylaminen, insbesondere hydroxy-substituierten Alkylaminen, wie Diethanolamin, Triethanolamin oder Tris-(hydroxymethyl)-aminomethan, Piperidin oder Morpholin.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und innerer Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solcher entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische und therapeutische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro

die nach Vitamin-A-Mangel einsetzende Keratinisierung auf. Diese Keratinisierung gehört zur Frühphase der Carcinogenese, die in einer ähnlichen Technik in vivo nach der Initiation durch chemische Verbindungen, durch energiereiche Strahlung oder nach viraler Zelltransformation durch die erfindungsgemäßen Verbindungen der Formel (I) inhibiert wird. Diese Methodik kann aus Cancer Res. 36, 964—972 (1976) oder aus Nature 250, 64—66 (1974) und Nature 253, 47—50 (1975) entnommen werden.

Darüber hinaus werden durch die erfindungsgemäßen Verbindungen die Proliferationsraten bestimmter maligner transformierter Zellen inhibiert. Vorzugsweise arbeitet man mit der S 91-Melanoma- oder der L 929-Zellinie. Diese Methodik kann aus J. Natl. Cancer Inst. 60, 1035—1041 (1978) und Experimental Cell Research 117, 15—22 (1978) entnommen werden. Die antiarthritische Wirkung der erfindungsgemäßen Verbindungen kann in üblicher Weise im Tierexperiment im Adjuvans-Arthritis-Modell bestimmt werden.

Die folgenden Angaben dienen zum Beleg der überlegenen Wirkung gegenüber Vitamin-A-säure.

### 1. Die Aufhebung der Keratinisierung in Trachealkulturen durch all-E-Retinsäure-N-(tetrazol-5-yl)-amid und 13-Z-Retinsäure-N-(tetrazol-5-yl)-amid zum Nachweis der Antitumorwirkung

Das Testmodell bestimmt die intrinsische Eigenschaft der erfindungsgemäßen Verbindungen, die Differenzierung von Epithelialzellen zu steigern. Die hohe Signifikanz dieser Screeningsmethode bei der Vorhersage der potentiellen Verwendung eines neuen Retinoids zur Prevention von Tumoren im Epithelgewebe wird allgemein anerkannt. Dabei ist bekannt, daß jedes in-vitro Testsystem mit Nachteilen behaftet ist, wenn es um die Vorhersage der in-vivo Aktivität geht. Abgesehen von diesen grundsätzlichen Einschränkungen ist das System der Trachealzellkulturen eine der wertvollsten Methoden, um die biologische Aktivität neuer Retinoide zu ermitteln.

Das Testmodell bestimmt die Fähigkeit der beiden Substanzen, die Keratinisierung in einem definierten in-vitro System aufzuheben. Tracheen von Hamstern, die sich in einem sehr frühen Zustand eines Vitamin A-Mangels befinden, wurden in Kutltur genommen. Zu diesem Zeitpunkt waren die Tiere 29 bis 30 Tage alt (nachdem man sie nach 21 Tagen entwöhnt hatte) und sie zeigten immer noch eine Gewichtszunahme. Das Durchschnittsgewicht betrug 47 bis 52 g. Das tracheale Epithel war im allgemeinen ein schwaches Säulen- oder Pflasterepithel mit nur vereinzelten Stellen squamöser Metaplasien. Jede Trachea wurde vom Kehlkopf bis zur Carina entlang der membranartigen Dorsalwand geöffnet und in serum-freiem Medium (CMRL-1066 ergänzt durch kristallines Rinderinsulin, 1,0 µg/ml, Hydrocortison-hemisuccinat, 0,1 µg/ml; Glutamin 2 mM; Penicillin, 100 Einheiten/ml und Streptomycin, 100 ug/ml) in Kultur genommen. Die Kulturen wurden mit einem Gemisch aus 50% Sauerstoff, 45% Stickstoff und 5% Kohlendioxid begast. Die Kulturschalen wurden um 35,5 bis 36,0 Grad geschwenkt, um den Kontakt der Tracheen mit Gas und Medium zu gewährleisten. Alle Tracheen wurden zunächst 3 Tage ohne Retinoid-Zusatz in dem Kulturmedium gehalten. Nach 3 Tagen wurden einige Tracheen entnommen. Sie zeigten fast alle deutliche squamöse Metaplasien. Die verbleibenden Tracheen wurden in Gruppen eingeteilt, die dann mit folgenden Zusätzen behandelt wurden:

a) Testsubstanz in spektroskopisch reinem Dimethylsulfoxid gelöst; die endgültige Konzentration an Dimethylsulfoxid im Kulturmedium war niemals größer als 0,1%.
b) Die äquivalente Menge Dimethylsulfoxid ohne weiteren Zusatz.

Das Nährmedium wurde dreimal wöchentlich gewechselt. Alle verbliebenen Tracheen wurden nach 10 Tagen in Kultur aufgearbeitet. Die Tracheen wurden in 10%iger gepufferter Formaldehydlösung fixiert und in Paraffin eingebettet. Schnitte von 5 µm durch das Zentrum wurden mit Hexatoxylin und Eosin angefärbt und unter dem Mikroskop auf die Anwesenheit von Keratin und Keratohyalin untersucht; beides wurde in etwa 90% aller ohne Testsubstanz gehaltenen Kontrollkulturen beobachtet. Dosis-Wirkungskurven der erfindungsgemäßen Verbindungen wurden aufgenommen. In der nachfolgenden Tabelle 1 werden die extrapolierten molaren Dosen angegeben, die in der Hälfte der Kulturen die Keratinisierung unterbinden (ED 50%).

Tabelle 1

| | $ED_{50}$ [Mol/l] | Zahl der Kulturen |
|---|---|---|
| all-E-Retinsäure-N-(tetrazol-5-yl)-amid | $<1 \times 10^{-10}$ | 65 |
| 13-Z-Retinsäure-N-(tetrazol-5-yl)-amid | $2 \times 10^{-10}$ | 63 |
| all-E-Retinsäure | $3 \times 10^{-11}$ | 65 |

2. Vergleichende Toxizität von all-E-Retinsäure-N-(tetrazol-5-yl)-amid und 13-Z-Retinsäure-N-(tetrazol-5-yl)-amid

Die erfindungsgemäßen Verbindungen sind bedeutend weniger toxisch als all-E-Retinsäure bei oraler Gabe an Ratten. Beide Retinoide wurden an Ratten in hohen Dosen verabreicht und die Behinderung des Wachstums und die Letalität über einen Zeitraum von zwei Wochen beobachtet (Tabelle 2).

a) Männliche Sprague-Dawley CD Ratten (Charles River, 50 bis 75 g) wurden statistisch verteilt und eine Woche täglich vor Versuchsbeginn gewogen. Die Tiere wogen zu Beginn der toxikologischen Untersuchung 95 bis 110 g.
b) Trägersubstanz für die Testsubstanzen ist Corn Oil, das 4% Chloroform enthält. Die Dosis ist 25 µmol an Prüfsubstanz in 0,5 ml des Trägermaterials pro Tag.

Tabelle 2

| | Anteil der noch lebenden Tiere | | Mittleres Gewicht*) | |
|---|---|---|---|---|
| | 6. Tag | 11. Tag | 6. Tag | 11. Tag |
| all-E-Retinsäure-N-(tetrazol-5-yl)-amid | 3/4 | 3/4 | $124 \pm 6$ | $155 \pm 4$ |
| 13-Z-Retinsäure-N-(tetrazol-5-yl)-amid | 2/5 | 2/5 | $122 \pm 18$ | $140 \pm 27$ |
| all-E-Retinsäure | 3/5 | 0/5 | $95 \pm 2$ | – |
| Unbehandelte Kontrollgruppe | 5/5 | 5/5 | $140 \pm 2$ | $182 \pm 2$ |

*) Mittelwerte mit Standardabweichungen.

3. Komedolytische Aktivität zum Nachweis der dermatologischen Aktivität

all-E-Retinsäure-N-(tetrazol-5-yl)-amid und 13-Z-Retinsäure-N-(tetrazol-5-yl)-amid wurden am Modell des Kaninchenohrs auf ihre komedolytische Aktivität untersucht.

Die Ausbildungen von Komedonen wurde durch einmalige topische Applikation (0,5 ml pro Tag) von 5% Teer in Polyan® (Ester von Lanolinalkohol und Linolensäure, Herst. Amerchol Corp., USA) auf beide Ohren von Albinokaninchen an 5 aufeinanderfolgenden Tagen pro Woche über zwei Wochen induziert. Anschließend erfolgte die topische Behandlung der Testsubstanzen in Alkohol: Propylenglykol (70 : 30, v/v; 0,5 ml) der inneren Hautoberfläche eines Ohres jedes Kaninchens einmal täglich an 5 aufeinanderfolgenden Tagen pro Woche über zwei Wochen. Das zweite Ohr jedes Tieres diente als unbehandelte Kontrolle.

Nach der sich noch anschließenden Behandlung (~72 Stunden) mit der Prüfsubstanz wurden die Kaninchen getötet. Eine Hautprobe von ungefähr 6 $cm^2$ aus jeder Ohrmuschel gerade außerhalb des Gehörganges wurde entfernt und diese in ca. 1 $cm^2$ große Scheiben zerteilt.

Diese Hautpartien wurden 2 Minuten in 60°C warmes Wasser getaucht. Nach dem vorsichtigen Abschälen der Epidermis mit dem flachen Ende eines Spatels und einer kleinen Pinzette brachte man sie mit der dermalen Seite nach oben auf Objektträger. Nach der Lufttrocknung über Nacht wurden die Prüfstreifen unter dem Stereomikroskop beurteilt. Follikulare Partien mit hornigem Material bleiben intakt. Die Komedone sind erkennbar als diskrete, gleichgestaltige, zylindrische bis runde

Hornteile, deren Größe und Zahl proportional zur Aktivität der geprüften Substanz ist. Der komedolytische Effekt wurde bestimmt als die Abnahme der Komedonenzahl im Vergleich zum Kontrollohr.

Tabelle 3

| | % (Konzentration) | N (Tierzahl) | % (Komedolytische Aktivität) |
|---|---|---|---|
| all-E-Retinsäure-N-(tetrazol-5-yl)-amid | 0,025 | 6 | 35,1 |
| 13-Z-Retinsäure-N-tetrazol-5-yl)-amid | 0,1 | 6 | 39,4 |
| all-E-Retinsäure | 0,05 | 6 | 58,3 |

Die Zahlenwerte zeigen, daß die erfindungsgemäßen Verbindungen eine ausgeprägte komedolytische Aktivität, wenn auch etwas geringer gegenüber Vitamin-A-säure, im Modellsystem des Kaninchenohrs aufweisen.

4. Verträglichkeitsstudie der Testsubstanzen nach topischer Applikation

Jeder Versuch erfolgte mit sechs weißen männlichen Neuseeland-Kaninchen. Bei jedem Testtier wurden jeweils ungefähr 6 $cm^2$ große Rückenpartien rasiert. Nach dem Lösen der Prüfsubstanzen in Alkohol: Propylenglykol (70 : 30, v/v) wurden 0,2 ml davon mit einer automatischen Mikropipette an neun aufeinanderfolgenden Tagen durch vorsichtiges Einreiben an einer bestimmten Stelle zweimal täglich in sechssütndigen Intervallen appliziert.

Alle Testflächen wurden am Anfang und vor jeder ersten Applikation am Morgen subjektiv auf Erytheme und Abschuppung beurteilt. Man benutzte eine numerisch abgestufte Skala von 0 bis 3 (0=keine Reaktion, 1=mild, 2=mittel, 3=schwer). Der Mittelwert an Erythemen-Bildung und Abschuppung gibt die relative Irritationsfähigkeit der Prüfsubstanzen im Vergleich zu all-E- und 13-Z-retinsäure und einer Kontrollprobe des Trägermaterials wieder.

Tabelle 4
9-Tage Studie zur dermalen Irritation nach mehrmaliger Applikation

| | % (Konzentration) | N (Tierzahl) | Erythmen-bildung (Mittelwert) | Abschuppung | Bewertung Erytheme | Bewertung Abschuppung |
|---|---|---|---|---|---|---|
| all-E-Retinsäure-N-(tetrazol-5-yl)-amid | 0,025 | 6 | 0,8 | 0 | mild | keine |
| 13-Z-Retinsäure-N-(tetrazol-5-)yl-amid | 0,1 | 6 | 0,5 | 0,1 | mild | keine |
| all-E-Retinsäure | 0,025 | 6 | 2,3 | 1,9 | mittel | mittel |
| 13-Z-Retinsäure | 0,1 | 6 | 3,0 | 3,0 | schwer | schwer |

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffs mit den an sich in solchen Präparaten üblichen festen oder flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1%iger Konzentration, bevorzugt in 0,001- bis 0,1%iger Konzentration und bei systemischer Anwendung vorzugsweise in einer Einzeldosis von 0,1 bis 50 mg enthalten und täglich in einer oder mehreren Dosierungen je nach Art und Schwere der Erkrankung verabreicht werden.

Üblicherweise verwendete pharmazeutische technische Hilfsstoffe sind beispielsweise für die lokale Anwendung Alkohole, wie Isopropanol, oxethyliertes Ricinusöl oder oxethyliertes hydriertes Ricinusöl, Polyacrylsäure, Clycerinmonostearat, Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann ggf. ein Antioxidationsmittel, beispielsweise Tocopherol, sowie butyliertes Hydroxyanisol oder butyliertes Hydroxytoluol oder geschmacksverbessernde Zusätze, Stabilisierungsmittel, Emulgiermittel, Gleitmittel usw. zugesetzt werden. Voraussetzung ist, daß alle bei der Herstellung pharmazeutische Zubereitung verwendeten Stoffe toxikologisch unbedenklich sind und mit den verwendeten Wirkstoffen verträglich sind.

Herstellung der erfindungsgemäßen Verbindungen

## Beispiel 1

### all-E-Retinsäure-N-(tetrazol-5-yl)-amid

Zu einer Suspension von 15,3 g wasserfreiem 5-Aminotetrazol und 15 ml Pyridin in 200 ml Tetrahydrofuran wird innerhalb von 10 Minuten bei ca. −10°C eine Lösung von 140 mMol all-E-Retinsäurechlorid in 500 ml Tetrahydrofuran zugetropft und anschließend noch ca. 20 Stunden bei Raumtemperatur gerührt. Anderntags wird die entstandene Suspension in ein Gemisch aus 2 Liter Wasser, 75 ml konzentrierte Salzsäure und 250 ml Ethanol eingerührt, der entstandene Niederschlag abfiltriert und zur abschließenden Fein-Reinigung aus einem Methylenchlorid/Tetrahydrofuran/Heptan-Gemisch umkristallisiert. Es verbleiben 27 g all-E-Retinsäure-N-(tetrazol-5-yl)-amid vom Schmp. 228—230° C.

$E^1_1$ 1129 bei 390 nm.

$C_{27}H_{29}ON_5$ (367)

| | | | |
|---|---|---|---|
| Ber.: | 68,6 C | 8,0 H | 19,1 N |
| Gef.: | 68,6 C | 7,7 H | 19,4 N |

## Beispiel 2

### 13-Z-Retinsäure-N-(tetrazol-5-yl)-amid

Gemäß Beispiel 1 werden aus 7,7 g 5-Aminotetrazol, 7,5 ml Pyridin in 200 ml Tetrahydrofuran und 70 mMol 13-Z-Retinsäurechlorid in 250 ml Tetrahydrofuran 11 g 13-Z-Retinsäure-N-(tetrazol-5-yl)-amid vom Schmp. 215—217° C erhalten.

$E^1_1$ 1116 bei 390 nm.

$C_{27}H_{29}ON_5$ (367)

| | | | |
|---|---|---|---|
| Ber.: | 68,6 C | 8,0 H | 19,1 N |
| Gef.: | 68,8 C | 7,9 H | 19,3 N |

## Beispiel 3

### all-E-Retinsäure-N-(tetrazol-5-yl)-amid

Eine Mischung von 3 g all-E-Retinsäure und 1,9 g N,N'-Carbonyldiimidazol in 50 ml N,N'-Dimethylformamid wird 15 Minuten vorsichtig auf dem Dampfbad erwärmt und danach 45 Minuten bei Raumtemperatur stehengelassen. Anschließend gibt man 1 g 5-Aminotetrazol hinzu, erwärmt 1 Stunde auf ca. 50° C und filtriert die erkaltete Mischung in 0,3 Liter Wasser. Die Mischung wird anschließend mit Salzsäure auf pH 1 eingestellt. Der abfiltrierte Feststoff wird — wie in Beispiel 1 beschrieben — auf analysenreines all-E-Retinsäure-N-(tetrazol-5-yl)-amid aufgearbeitet.

## Beispiel 4

### all-E-Retinsäure-N-(tetrazol-5-yl)-amid-Natriumsalz

Zu einer Suspension von 1 g all-E-Retinsäure-N-(tetrazol-5-yl)-amid in 200 ml Wasser und 30 ml Tetrahydrofuran gibt man ungefähr 1 Mol-Äquivalent wäßrige Natriumhydroxid-Lösung. Nach mehrstündigem Rühren wird die Reaktionsmischung filtriert. Man erhält das als Titelverbindung genannte Salz mit einem Gehalt an Wasser durch Gefriertrocknung des Filtrats.

Ersetzt man bei dieser Arbeitsweise Natriumhydroxid durch andere Basen, beispielsweise durch Ethanolamin, Ethylendiamin, Diethanolamin, Triethanolamin oder Tris-(hydroxymethyl)-aminomethan, erhält man die entsprechenden Aminsalze, die teilweise ohne Substanzisolierung als wäßrige Lösungen verwendbar sind.

Geeignete pharmazeutische Zubereitungen oder Arzneistoffträger für die äußerliche Anwendung sind z. B.:

## Beispiel I

Lösung

| | | |
|---|---|---|
| all-E-Retinsäure-N-(tetrazol-5-yl)-amid-Na-Salz | | 0,25 g |
| oxethyliertes hydriertes Ricinusöl (Cremophor RH 40, Hersteller BASF AG, Ludwigshafen) | | 35,0 g |
| Polyäthylenglykol 400 | | 35,0 g |
| oxethyliertes Ricinusöl (Softigen 767, Hersteller Chemische Werke, Witten) | | 10,0 g |
| demineralisiertes Wasser | ad | 100,0 g |

Cremophor RH 40 und Softigen 767 werden gemischt und auf 70° C erhitzt. Die Wirksubstanz wird unter Rühren gelöst und Polyethylenglykol 400 zugesetzt. Die Lösung wird dann langsam auf 40° C erhitztes Wasser zugesetzt. Die fertige Lösung wird filtriert und in z. B. 100 ml Flaschen gefüllt.

## Beispiel II

Creme

| | | |
|---|---|---|
| all-E-Retinsäure-N-(tetrazol-5-yl)-amid | | 0,1 g |
| Butylhydroxytoluol | | 0,1 g |
| Glycerinmonostearat | | 11,0 g |
| Polyethylenglykol 400-Stearat | | 6,0 g |
| ethoxylierter Fettalkohol | | 4,0 g |
| Paraffinöl | | 10,0 g |
| p-Hydroxybenzoesäureester (Nipasteril, Hersteller Nipalaboratorium, Hamburg) | | 0,2 g |
| Parfumöl | | 0,1 g |
| demineralisiertes Wasser | ad | 100,0 g |

Die Fette wurden geschmolzen und die feinstgepulverte Wirksubstanz sowie Butylhydroxytoluol unter Rühren bei 65° C darin verteilt (Lösung I). Das Wasser wird mit dem Nipaester aufgekocht und auf 65° C abgekühlt (Lösung II). In kleinen Anteilen wird Lösung II unter gutem Rühren in Lösung I einemulgiert. Nach dem Abkühlen auf 45° C wird das Parfumöl zugesetzt und die Emulsion unter Rühren auf Zimmertemperatur abgekühlt. Die fertige Creme wird in innenschutzlackierte Tuben abgefüllt.

Beispiel III

Gel

| | | |
|---|---|---|
| all-E-Retinsäure-N-(tetrazol-5-yl)-amid | | 0,01 g |
| Butylhydroxytoluol | | 0,1 g |
| oxethyliertes Ricinusöl (Cremophor El, Hersteller BASF AG, Ludwigshafen) | | 35,0 g |
| Isopropanol | | 20,0 g |
| Polyacrylsäure (Carbopol, Hersteller Goodrich, Hamburg) | | 1,5 g |
| Triethanolamin | | 0,002 g |
| p-Hydroxybenzoesäureester (Nipasteril, Hersteller Nipalaboratorium, Hamburg) | | 0,2 g |
| demineralisiertes Wasser | ad | 100,0 g |

Das Cremophor El wird auf 60°C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in dem Wasser verteilt (Lösung II). Lösung II wird unter gutem Rühren in kleinen Anteilen zu Lösung I gemischt. Der pH-Wert des Gemisches wird mit Triethanolamin auf 4,5 eingestellt. Das fertige Gel wird in innenschutzlackierte Tuben abgefüllt.

Für die systemische Anwendung besonders geeignete Zubereitungen oder Arzneistoffträger sind z. B.:

Beispiel IV

Tropfen

| | | |
|---|---|---|
| 13-Z-Retinsäure-N-(tetrazol-5-yl)-amid | | 0,1 g |
| Propylenglykol | | 25,0 g |
| Ethylalkohol | ad | 50,0 g |

Ethylalkohol und Propylenglykol werden miteinander gemischt und die Wirksubstanz unter Erwärmen auf 35°C und unter Rühren gelöst. Nach Filtration wird die Lösung in dunkle Tropfflaschen abgefüllt.

Beispiel V

Hartgelatinekapseln

| | | |
|---|---|---|
| 13-Z-Retinsäure-N-(tetrazol-5-yl)-amid | | 1 mg |
| Milchzucker | ad | 0,25 g |

Die Bestandteile werden gesiebt, gemischt und auf einer geeigneten Kapselfüll- und -verschlußmaschine in Hartgelatinekapseln der Größe 2 gefüllt.

**Patentansprüche**

1. Verbindungen der Formel (I),

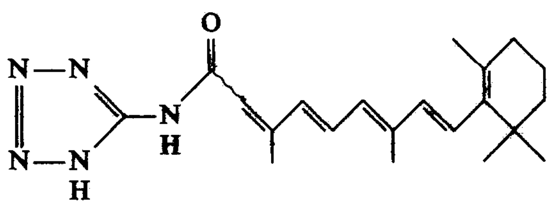

(I)

in der die geschweifte Bindung zwischen den Kohlenstoffatomen 14 und 15 eine all-E- oder 13-Z-Verknüpfung bedeutet, und ihre physiologisch verträglichen Salze.

2. all-E-Retinsäure-N-(tetrazol-5-yl)-amid und dessen physiologisch verträglichen Salze.

3. 13-Z-Retinsäure-N-(tetrazol-5-yl)-amid und dessen physiologisch verträglichen Salze.

4. Verfahren zur Herstellung von Verbindungen der Formel (I) nach Anspruch 1, dadurch gekennzeichnet, daß man ein reaktionsfähiges Retinsäurederivat der Formel (II)

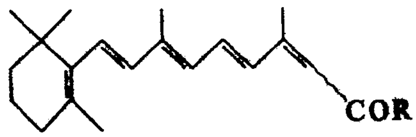

(II)

in der die geschweifte Bindung zwischen den Kohlenstoffatomen 14 und 15 eine all-E- oder 13-Z-Verknüpfung bedeutet, und R für eine geeignete Abgangsgruppe steht, zweckmäßigerweise in einem Lösungsmittel und ggf. in Gegenwart eines säurebindenden Mittels in an sich bekannter Weise mit 5-Aminotetrazol umsetzt und ggf. die erhaltene Verbindung mit einer Base in ein physiologisch verträgliches Salz überführt
oder daß man all-E- oder 13-Z-Retinsäure der Formel (III)

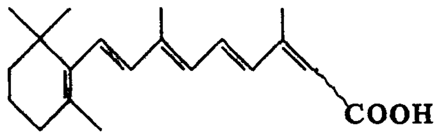

(III)

in Gegenwart eines die Carboxylgruppe aktivierenden wasserabspaltenden Mittels in einem Lösungsmittel mit 5-Aminotetrazol kondensiert und ggf. die erhaltene Verbindung mit einer Base in ein physiologisch verträgliches Salz überführt.

5. Therapeutisches Mittel, enthaltend neben üblichen Träger- und Verdünnungsmitteln eine Verbindung der Formel (I) oder ihr physiologisch verträgliches Salz als Wirkstoff.

6. Eine Verbindung der Formel I nach Anspruch 1 zur topischen und systemischen Verwendung bei der Bekämpfung von Neoplasien, Akne und Dermatosen.

**Claims**

1. A compound of the formula (I)

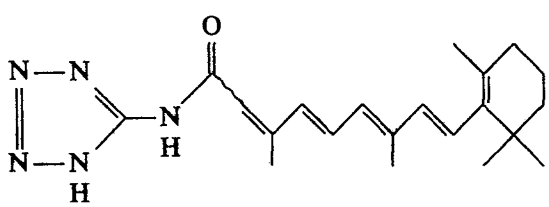

(I)

where the squiggly bond between carbon atoms 14 and 15 is an all-E-link or 13-Z-link, and its physiologically tolerated salts.

2. all-E-N-(tetrazol-5-yl)-retinamide and its physiologically tolerated salts.

3. 13-Z-N-(tetrazol-5-yl)-retinamide and its physiologically tolerated salts.

4. A process for the preparation of a compound of the formula (I) as claimed in claim 1, wherein a reactive retinoic acid derivative of the formula (II)

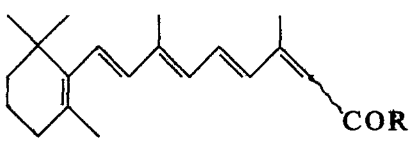

(II)

where the squiggly bond between carbon atoms 14 and 15 is an all-E-link or 13-Z-link and R is a suitable leaving group, is reacted, in a conventional manner, with 5-amino-tetrazole, advantageously in a solvent and in the presence or absence of an acid acceptor, and, if desired, the resulting compound os converted into a physiologically tolerated salt by means of a base,
or wherein all-E-retinoic acid or 13-Z-retinoic acid of the formula (III)

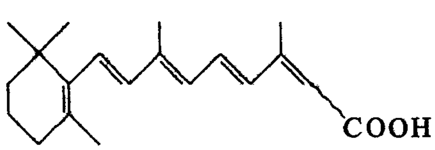

(III)

is condensed with 5-aminotetrazole in a solvent in the presence of a dehydrating agent which activates the carboxyl group, and, if desired, the resulting compound is converted into a physiologically tolerated salt by means of a base.

5. A therapeutic agent containing a compound of the formula (I), or a physiologically tolerated salt thereof, as active ingredient, in addition to conventional vehicles and diluents.

6. A compound of the formula (I) as claimed in claim 1, for topical and systemic use in the treatment of neoplasias, acne and dermatoses.

**Revendications**

1. Composés de la formule (I)

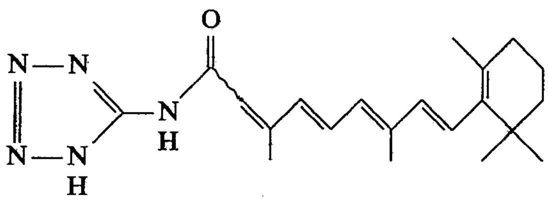

(I)

dans laquelle la liaison ondulée entre les atomes de carbone 14 et 15 représente une liaison allo-E ou 13-Z, et leurs sels physiologiquement compatibles.

2. Le N-(tétrazol-5-yl)-amide de l'acide allo-E-rétinoique.

3. Le N-(tétrazol-5-yl)-amide de l'acide 13-Z-rétinoique.

4. Procédé de préparation de composés de la formule (I) selon la revendication 1, caractérisé en ce que l'on fait réagir un dérivé réactif de l'acide rétinoique de la formule (II)

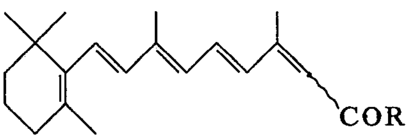

(II)

dans laquelle la liaison ondulée entre les atomes de carbone 14 et 15 représente une liaison allo-E ou 13-Z et R désigne un groupe clivable approprié, de mantière connue en soi, de préférence dans un solvant et éventuellement en présence d'un agent fixant les acides, avec le 5-amino-tétrazole, le composé formé étant le cas échéant transformé par une base en un sel physiologiquement compatible,
ou l'on condense l'acide allo-E- ou 13-Z-rétinoique de la formule (III)

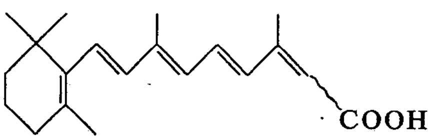

(III)

dans un solvant, en présence d'un agent déshydratant activant le groupe carboxyle, et le 5-amino-tétrazole et on transforme le cas échéant le composé obtenu avec une base en un sel physiologiquement compatible.

5. Composition thérapeutique, contenant à côté de véhicules et diluants usuels un composé de la formule (I) ou un sel physiologiquement compatible de celui-ci en tant que principe actif.

6. Composé de la formule (I) selon la revendication 1 pour l'utilisation topique ou systémique dans le traitement de néoplasmes, de l'acné et de dermatoses.